# EUROPÄISCHE PATENTSCHRIFT

(11) **EP 0 854 707 B1**
(45) Veröffentlichungstag und Bekanntmachung des Hinweises auf die Patenterteilung: **27.11.2002**
(21) Anmeldenummer: 96934494.4
(22) Anmeldetag: 30.09.1996
(51) Int. Cl.: A61K 9/20

(54) **FESTE ARZNEIFORMEN, ERHÄLTLICH DURCH EXTRUSION EINER ISOMALT ENTHALTENDEN POLYMER-WIRKSTOFF-SCHMELZE**
SOLID MEDICAMENTS OBTAINED BY EXTRUSION OF AN ISOMALT-CONTAINING POLYMER-ACTIVE SUBSTANCE MELT
FORMES MEDICAMENTEUSES SOLIDES OBTENUES PAR EXTRUSION D'UNE MASSE FONDUE DE SUBSTANCE ACTIVE POLYMERE CONTENANT DE L'ISOMALT

(30) Priorität: 29.09.1995 DE 19536394
(43) Veröffentlichungstag der Anmeldung: 29.07.1998
(73) Patentinhaber: BASF AKTIENGESELLSCHAFT, 67056 Ludwigshafen (DE)
(72) Erfinder: ZEIDLER, Jürgen, D-67112 Mutterstadt (DE); ROSENBERG, Joerg, D-67158 Ellerstadt (DE); NEUMANN, Jörg, D-67117 Limburgerhof (DE); BREITENBACH, Jörg, D-68199 Mannheim (DE)
(86) Internationale Anmeldenummer: EP9604262
(87) Internationale Veröffentlichungsnummer: WO97012603

(56) Entgegenhaltungen:
- EP-A- 0 354 442
- WO-A-95/34293

## Beschreibung

Die vorliegende Erfindung betrifft feste Arzneiformen, erhältlich durch Extrusion und anschließender Formgebung einer lösungsmittelfreien Schmelze, enthaltend neben einem oder mehreren Wirkstoffen
A) 2 bis 90 Gew.-% eines thermoplastisch verarbeitbaren Polymers,
B) 5 bis 89,9 Gew.-% Isomalt, und
C) 0 bis 5 Gew.-% Lecithin,
wobei die angegebenen Mengen auf das Gesamtgewicht der Arzneiform bezogen sind.

Weiterhin betrifft die Erfindung ein Verfahren zur Herstellung solcher Arzneiformen.

Wirkstoffhaltige Zubereitungen, die durch Schmelzextrusion hergestellt werden, sind allgemein bekannt.

Das Extrudieren von wirkstoffhaltigen Schmelzen wasserlöslicher Polymeren, vorzugsweise von Copolymeren des Vinylpyrrolidons, ist beispielsweise in der EP-A 240 904 und der EP-A 240 906 beschrieben.

Das Verfahren der Schmelzextrusion ist für eine Vielzahl von Wirkstoffen anwendbar. Durch Einsatz unterschiedlicher Hilfsstoffe können die Eigenschaften der hergestellten Formulierungen wie beispielsweise die Auflösegeschwindigkeit der Arzneiform im Gastrointestinaltrakt gezielt beeinflußt werden.

Will man schnell freisetzende, feste Arzneiformen herstellen, so muß man Hilfsstoffe mit großer Lösegeschwindigkeit nach entsprechend hoher Wasserlöslichkeit verwenden, die zudem die thermoplastische Verarbeitbarkeit der wirkstoffhaltigen Polymerschmelze nicht beeinträchtigen dürfen. Bisher wurden dazu im allgemeinen Zuckeralkohole wie Mannitol oder Sorbitol oder Zucker wie beispielsweise Lactose eingesetzt.

Nachteilig an bekannten Zusammensetzungen ist aber, daß diese teilweise eine schlechte Verarbeitbarkeit, verursacht durch eine starke Klebeneigung bei der Formgebung, vor allem bei der Kalandrierung aufweisen. Außerdem lassen diese Zusammensetzungen häufig noch hinsichtlich der Freisetzungsgeschwindigkeit zu wünschen übrig. Hinzu kommt, daß auch die mangelnde mechanische Belastbarkeit der Tabletten wegen starker Versprödung und damit auftretender Rißbildung noch Anlaß zu Verbesserungen gibt.

Aufgabe der vorliegenden Erfindung war es, Arzneiformen zu finden, die eine schnelle Wirkstoff-Freisetzung bei gleichzeitig sehr guter Verarbeitbarkeit und guter Stabilität der Arzneiform aufweisen.

Demgemäß wurden die eingangs definierten Arzneiformen gefunden.

Als Wirkstoffe kommen erfindungsgemäß alle solchen in Betracht, die unter den Verarbeitungsbedingungen der Schmelzextrusion eine ausreichende thermische Stabilität aufweisen.

### Geeignete Wirkstoffe sind beispielsweise

Acebutolol, Acetylcysteine, Acetylsalicylsäure, Aciclovir, Albrazolam, Alfacalcidol, Allantoin, Allopurinol, Ambroxol, Amikacin, Amiloride, Aminoacetic Acid, Amiodarone, Amitriptyline, Amlodipine, Amoxicillin, Ampicillin, Ascorbic Acid, Aspartam, Astemizole, Atenolol, Beclometasone, Benserazide, Benzalkonium Hydrodrochlorid, Benzocaine, Benzoesäure, Betametasone, Bezafibrate, Biotin, Biperiden, Bisoprolol, Bromacepam, Bromhexine, Bromocriptine, Budesonide, Bufexamac, Buflomedil, Buspirone, Caffeine, Camphor, Captopril, Carbamazepine, Carbidopa, Carboplatin, Cefaclor, Cefalexin, Cefatroxil, Cefazolin, Cefixime, Cefotaxime, Ceftazidine, Ceftriaxone, Cefuroxime, Celediline, Chloramphenicol, Chlorhexidine, Chlorpheniramine, Chlortalidone, Choline, Ciclosporin, Cilastatin, Cimetidine, Ciprofloxacin, Cisapride, Cisplatin, Clarithromycin, Clavulanic Acid, Clomibramine, Clonazepam, Clonidine, Clotrimazole, Codeine, Colestyramine, Cromoglicinsäure, Cyanocobalamin, Cyproterone, Desogestrel, Dexamethasone, Dexpanthenol, Dexthromethorphan, Dextropropoxiphene, Diazepam, Diclofenac, Digoxin, Dihydrocodeine, Dihydroergotamine, Dihydroergotoxin, Diltiazem, Diphenhydramine, Dipyridamole, Dipyrone, Disopyramide, Domperidone, Dopamine, Doxocyclin, Enalapril, Ephedrine, Epinephrine, Ergocalciferol, Ergotamine, Erythromycin, Estradiol, Ethinylestradinol, Etoposide, Eucalyptus Globulus, Famotidine, Felodipine, Fenofibrate, Fenoterol, Fentanyl, Flavin Mononucleotide, Fluconazole, Flunarizine, Fluorouracil, Fluoxetine, Flurbiprofen, Furosemide, Gallopamil, Gemfibrozil, Gentamincin, Ginkgo Biloba, Glibenclamid, Glipizide, Glozapine, Glycyrrhiza Glabra, Griseofulvin, Guaifenesin, Haloperidol, Heparin, Hyaluronsäure, Hydrochlorothiazide, Hydrocodone, Hydrocortisone, Hydromorphon, Ibratropium Hydroxide, Ibuprofen, Imipenem, Indomethacin, Iohexol, Iopamidol, Isosorbide Dinitrate, Isosorbide Mononitrate, Isotretionin, Ketotifen, Ketoconazole, Ketoprofen, Ketorolac, Labatalon, Lactulose, Lecithin, Levocarnitine, Levodopa, Levoglutamide, Levonorgestrel, Levothyroxine, Lidocaine, Lipase, Lipramin, Lisinopril, Loperamide, Lorazepam, Lovastatin, Medroxyprogesterone, Menthol, Methotrexate, Methyldopa, Methylprednisolone, Metoclopramide, Metoprolol, Miconazole, Midazolam, Minocycline, Minoxidil, Misoprostol, Morphine, Multivitamin and Minerals, N-Methylephedrine, Naftidrofuril, Naproxen, Neomycin, Nicardipine, Nicergoline, Nicotinamide, Nicotine, Nicotinic Acid, Nifedipine, Nimodipine, Nitrazepam, Nitrendipine, Nizatidine, Norethisterone, Norfloxacin, Norgestrel, Nortriptyline, Nystatin, Ofloxacin, Omeprazole, Ondansetron, Pancreatin, Panthenol, Pantothenic Acid, Paracetamol, Penicillin G, Penicillin V, Phenobarbital, Phenoxifylline, Phenoxymethylpenicillin, Phenylephrine, Phenylpropanolamin, Phenytoin, Piroxicam, Polymyxin B, Povidone-Iod, Pravastatin, Prazepam, Prazosin, Prednisolone, Prednison, Promocriptine, Propafenone, Propranolol, Proxyphyllin, Pseudoephedrine, Pyridoxine, Quinidine, Ramipril, Ranitidine, Reserpine, Retinol, Riboflavin, Rifampicin, Rutoside, Saccharin, Salbutamol, Salcatonin, Salicyl Acid, Simvastatin, Somatropin, Sotalol, Spironolactone, Sucralfate, Sulbactam, Sulfamethoxazole, Sulfasalazin, Sulpiride, Tamoxifen, Tegafur, Teprenone, Terazosin, Terbutaline, Terfenadine, Tetracyclin, Theophylline, Thiamine, Ticlopidine, Timolol, Tranexamsäure, Tretinoin, Triamcinolone Acetonide, Triamterene, Trimethoprim, Troxerutin, Uracil, Valproic Acid, Vancomycin, Verapamil, Vitamine E, Volinic Acid, Zidovudine.

Insbesondere eignen sich erfindungsgemäß Wirkstoffe, welche zur Herstellung schnellfreisetzender Formen (Instant-Release-Formen) geeignet sind.

Bevorzugter Wirkstoff ist Verapamil oder dessen physiologisch verträgliche Salze, besonders bevorzugt Verapamil-Hydrochlorid. Weiterhin bevorzugt ist Paracetamol.

Als thermoplastisch verarbeitbare, wasserlösliche Polymerkomponenten A) seien genannt:
- Alkylcellulosen wie Methylcellulose
- Hydroxyalkylcellulosen wie Hydroxymethyl-, Hydroxyethyl-, Hydroxypropyl- und Hydroxybutylcellulose,
- Hydroxyalkylmethylcellulosen wie Hydroxyethylmethyl- und Hydroxypropylmethylcellulose,
- Polyvinylpyrrolidon,
- Copolymere aus N-Vinylpyrrolidon und Vinylacetat mit bis zu 50 Gew.-% Vinylacetat,
- Carboxyalkylcellulosen wie Carboxymethylcellulosen,
- Polysaccharide wie Alginsäure und deren Alkali- und Ammoniumsalze,
- Polyethylenglykole
sowie Gemische solcher wasserlöslichen Polymeren.

Die Komponente A) soll in der Gesamtmischung aller Komponenten im Bereich von 50 bis 180°C, vorzugsweise 80 bis 140°C erweichen oder schmelzen, so daß die Masse extrudierbar ist. Gegebenenfalls kann eine Verarbeitbarkeit bei diesen Temperaturen durch Zugabe von Weichmachern erzielt werden.

"Wasserlöslich" bedeutet, daß sich in 100 g Wasser von 20°C mindestens 0,5 g, vorzugsweise mindestens 2 g des Polymeren lösen, gegebenenfalls auch kolloidal.

Bevorzugte Polymerkomponenten A) sind neben Polyvinylpyrrolidon die Polyethylenglykole und besonders bevorzugt ein Copolymer, welches durch radikalische Polymerisation von 60 Gew.-% N-Vinylpyrrolidon und 40 Gew.-% Vinylacetat erhalten wird. Bevorzugt sind auch Hydroxypropylcellulosen.

Als Hilfsstoff enthalten die Arzneiformen Isomalt, das auch unter dem Markennamen Palatinit® bekannt ist. Isomalt ist eine hydrierte Isomaltulose, die in etwa aus gleichen Teilen der Isomeren 1-O-a-D-Glucopyranosyl-D-mannit Dihydrat (1,1-GPM Dihydrat) und 6-O-α-D-Glucopyranosyl-D-Sorbit (1,6-GPS) besteht.

Die Korngröße des Isomalts kann in weiten Bereichen variieren, bevorzugt sind Korngrößen im Bereich von 0,1 bis 0,8 mm.

Das kommerziell erhältliche Isomalt wird hergestellt, indem in einem ersten Schritt Saccharose enzymatisch zu Isomaltulose (6-O-α-D-Glucopyranosyl-D-Fructose) umgelagert wird und anschließend diese Isomaltulose mit Wasserstoff/Raney-Nickel hydriert wird.

Die erfindungsgemäßen Arzneiformen enthalten die Polymerkomponenten A) in Mengen von 2 bis 90 Gew.-%, bevorzugt 10 bis 90 Gew.-%, besonders bevorzugt 10 bis 50 Gew.-%.. Isomalt wird in Mengen von 5 bis 89,9 Gew.-%, bevorzugt 10 bis 70 Gew.-%, besonders bevorzugt 20 bis 60 Gew.-%, eingesetzt.

Die Menge des Wirkstoffs richtet sich auch nach der therapeutischen Wirksamkeit. Er kann in Mengen von 0,1 bis 70, bevorzugt 10 bis 60 Gew.-%, besonders bevorzugt 20 bis 50 Gew.-% eingearbeitet werden.

Die Mengenangaben beziehen sich jeweils auf das Gesamtgewicht der Arzneiform (= 100 Gew.-%).

Zur weiteren Verbesserung der Verarbeitungseigenschaften können die Arzneiformen noch bis zu 5 Gew.-%, bevorzugt 2 bis 5 Gew.-% Lecithin enthalten.

Die erfindungsgemäßen Zubereitungen können weiterhin die üblichen pharmazeutischen Hilfsstoffe wie Füllstoffe, Schmiermittel, Formentrennmittel, Fließregulierungsmittel, Weichmacher, Farbstoffe und Stabilisatoren in Mengen bis zu ca. 50 Gew.-% enthalten.

Um die erfindungsgemäßen Arzneiformen herzustellen, kann die Wirkstoffkomponente entweder direkt in Form einer physikalischen Mischung mit den Polymeren A) verschmolzen werden oder mit der bereits vorliegenden Polymerschmelze gemischt werden.

Im übrigen erfolgt die Vermischung der Wirkstoffe mit der Schmelze in an sich bekannter Weise in Extrudern, vorzugsweise in Ein- oder Doppelschneckenextrudern in einem Temperaturbereich zwischen 50 und 180°C, bevorzugt 80 bis 140°C. Die Formgebung der wirkstoffhaltigen Polymerschmelze zu den erfindungsgemäßen Zubereitungen kann beispielsweise durch Kalandrierung des Extrudates nach der in der EP-A 240 906 beschriebenen Methode sowie nach dem aus der DE-A 38 30 355 bekannten Verarbeitungsverfahren durch Zerkleinerung des Extrudates mit rotierenden Messern in volumengleiche - noch verformbare - Stücke mit erstarrter Oberfläche und anschließendes Verpressen zu Tabletten in den üblichen Tablettiermaschinen erfolgen. Man kann so erhaltene Pellets nach Abrunden auch in Harzgelatinekapseln abfüllen.

Es ist möglich, die Hilfsstoffe in die Schmelze oder Lösung aus Wirkstoffen und Polymeren A) zu mischen. Ferner können die Hilfsstoffe zusammen mit dem Wirkstoff in die Polymerschmelze eingearbeitet werden. Außerdem können Gemische aus Hilfsstoffen, dem Wirkstoff und den Polymeren direkt verschmolzen werden. Im allgemeinen ist es üblich, eine physikalische Mischung aus Hilfsstoffen, Wirkstoffen und den Polymeren gemeinsam zu verschmelzen.

Die erfindungsgemäßen Zubereitungen werden als Arzneimittel verwendet und in Form von Tabletten, Pellets, Granulaten oder Kapseln oder anderen oral applizierbaren Arzneiformen eingesetzt. Vorzugsweise werden mit dem erfindungsgemäßen Zubereitungen Arzneiformen mit schneller Wirkstofffreisetzung hergestellt.

Falls gewünscht, kann die feste pharmazeutische Form auch mit einem üblichen Überzug zur Verbesserung des Aussehens und/oder des Geschmacks (Dragee) versehen werden, oder nach dem Filmcoating-Verfahren durch Aufsprühen einer wässrigen oder organischen Polymerlösung oder Dispersion zu Filmtabletten verarbeitet werden.

Durch die Zusammensetzung der erfindungsgemäßen Arzneiformen kann nicht nur eine schnelle Freisetzung des Wirkstoffs erzielt werden, sondern auch eine hervorragende Verarbeitbarkeit.

Die besonders bei zum Kleben neigenden Wirkstoffen, wie beispielsweise dem Verapamil-Hydrochlorid auftretenden Probleme bei der Handhabung der Schmelze und bei der Formgebung werden durch die erfindungsgemäßen Zubereitungen vermieden.

Die in den nachstehenden Beispielen beschriebenen Arzneiformen wurden gemäß dem in der EP-B 240 906 beschriebenen Kalandierverfahren erhalten. Sie wiesen eine gute mechanische Belastbarkeit auf und neigten nicht zu Klebrigkeit.

### Beispiele 1 bis 7

Extrusion/Kalandrierung - Allgemeines:
Extrudertyp: Zweischneckenextruder
Anzahl der Schüsse: 4 + Kopf (Düse)
Tablettenformen: rund, Linsenform, 300 mg schwer
Massenfluß: 20 - 25 kg/h
Bestimmung der Wirkstoff-Freisetzung
Paddle-Methode - USP XXIII (Verapamil-HCl tablets) Seite 1625 Drehzahl: 50 upm
Paddle-Medium: 0,1 mol/l HCl
Als Polymer A) wurde ein Copolymerisat hergestellt aus 60 Gew.-% N-Vinylpyrrolidon und 40 Gew.-% Vinylacetat ("Copolyvidon" nach DAB) verwendet.

Die Mengenangaben beziehen sich jeweils auf Gew.-%.

### Beispiel 1 (Vergleichsbeispiel)

| | |
|---|---|
| Verapamil-HCl | 26,67 % |
| Copolyvidon | 40,00 % |
| Mannitol | 28,33 % |
| Lecithin | 5,00 % |
| Gesamtmasse | 363 mg |

### Extrusionsbedingungen:

| | Schuß 1 | Schuß 2 | Schuß 3 | Schuß 4 | Kopf |
|---|---|---|---|---|---|
| Temp. (°C) | 80 | 100 | 115 | 115 | 115 |

Die Prüfung der Wirkstoff-Freisetzung ergab, daß nach 30 min 52,4 % des Wirkstoffs freigesetzt war.

### Beispiel 2

| | |
|---|---|
| Verapamil-HCl | 26,67 % |
| Copolyvidon | 40,00 % |
| Isomalt | 28,33 % |
| Lecithin | 5,00 % |
| Gesamtmasse | 368 mg |

### Extrusionsbedingungen:

| | Schuß 1 | Schuß 2 | Schuß 3 | Schuß 4 | Kopf |
|---|---|---|---|---|---|
| Temp. (°C) | 100 | 120 | 130 | 130 | 130 |

Die Prüfung der Wirkstoff-Freisetzung ergab, daß nach 30 min 91,5 % des Wirkstoffs freigesetzt war.

### Beispiel 3

| | |
|---|---|
| Verapamil-HCl | 33,33 % |
| Copolyvidon | 31,67 % |
| Isomalt | 32,00 % |
| Lecithin | 3,00 % |
| Gesamtmasse | 242 mg |

### Extrusionsbedingungen:

| | Schuß 1 | Schuß 2 | Schuß 3 | Schuß 4 | Kopf |
|---|---|---|---|---|---|
| Temp. (°C) | 80 | 100 | 110 | 110 | 110 |

Die Prüfung der Wirkstoff-Freisetzung ergab, daß nach 20 min 65,4 % des Wirkstoffs freigesetzt war.

### Beispiel 4

| | |
|---|---|
| Verapamil-HCl | 33,33 % |
| Copolyvidon | 23,67 % |
| Isomalt | 40,00 % |
| Lecithin | 3,00 % |
| Gesamtmasse | 246 mg |

### Extrusionsbedingungen:

| | Schuß 1 | Schuß 2 | Schuß 3 | Schuß 4 | Kopf |
|---|---|---|---|---|---|
| Temp. (°C) | 80 | 100 | 110 | 110 | 110 |

Die Prüfung der Wirkstoff-Freisetzung ergab, daß nach 20 min 77,0 % des Wirkstoffs freigesetzt war.

### Beispiel 5

| | |
|---|---|
| Verapamil-HCl | 33,33 % |
| Copolyvidon | 13,67 % |
| Isomalt | 50,00 % |
| Lecithin | 3,00 % |
| Gesamtmasse | 252 mg |

### Extrusionsbedingungen:

| | Schuß 1 | Schuß 2 | Schuß 3 | Schuß 4 | Kopf |
|---|---|---|---|---|---|
| Temp. (°C) | 80 | 100 | 110 | 110 | 110 |

Die Prüfung der Wirkstoff-Freisetzung ergab, daß nach 20 min 85,8 % des Wirkstoffs freigesetzt war.

### Beispiel 6

| | |
|---|---|
| Verapamil-HCl | 33,33 % |
| Copolyvidon | 13,67 % |
| Isomalt | 50,00 % |
| Lecithin | 3,00 % |
| Gesamtmasse | 252 mg |

### Extrusionsbedingungen:

| | Schuß 1 | Schuß 2 | Schuß 3 | Schuß 4 | Kopf |
|---|---|---|---|---|---|
| Temp. (°C) | 80 | 100 | 110 | 110 | 110 |

Die Prüfung der Wirkstoff-Freisetzung ergab, daß nach 30 min 93,2 % des Wirkstoffs freigesetzt war.

### Beispiel 7 (Vergleichsbeispiel)

| | |
|---|---|
| Verapamil-HCl | 30,00 % |
| Copolyvidon | 70,00 % |

### Extrusionsbedingungen:

| | Schuß 1 | Schuß 2 | Schuß 3 | Schuß 4 | Kopf |
|---|---|---|---|---|---|
| Temp. (°C) | 90 | 130 | 130 | 140 | 140 |

Die Prüfung der Wirkstoff-Freisetzung ergab, daß nach 30 min 93,7 % des Wirkstoffs freigesetzt war.

### Beispiel 8

| | |
|---|---|
| Verapamil-HCl | 32 % |
| Isomalt | 41,5 % |
| Copolyvidon | 24 % |
| Hydriertes Rhizinusöl | 2,00 % |
| Hochdisperses Siliciumdioxid | 0,5 % |

| | Schuß 1 | Schuß 2 | Schuß 3 | Schuß 4 | Kopf |
|---|---|---|---|---|---|
| Temp. (°C) | 80 | 100 | 110 | 110 | 110 |

Die Prüfung der Wirkstoff-Freisetzung ergab, daß nach 30 min mindestens 96,7 % des Wirkstoffes freigesetzt war.

### Beispiele 9 und 10

### Paracetamol - Tabletten

Extrusion / Kalandrierung - Allgemeines
Extrudertyp: Zweischneckenextruder
Tablettenform: oblong, 700 mg schwer
Massenfluß: 20 - 25 kg/h
Paddle-Methode: USP 23 (Paracetamol Tablets)

Die Mengenangaben beziehen sich jeweils auf Gew.-%.

### Beispiel 9

| | |
|---|---|
| Paracetamol | 72 % |
| Polyvinylpyrrolidon, K-Wert 30 | 4 % |
| Isomalt | 17,25 % |
| Hochdisperses Siliciumdioxid | 1 % |
| Natriumstärkeglykolat | 5 % |
| Natriumlaurylsulfat | 0,75 % |

| | Schuß 1 | Schuß 2 | Schuß 3 | Schuß 4 | Kopf |
|---|---|---|---|---|---|
| Temp. (°C) | 80 | 120 | 120 | 130 | 145 |

Die Prüfung der Wirkstoff-Freisetzung ergab, daß nach 30 min 100 % des Wirkstoffes freigesetzt war. Paddle-Methode.

### Beispiel 10

| | |
|---|---|
| Paracetamol | 72 % |
| Copolyvidon | 4 % |
| Isomalt | 17,25 % |
| Hochdisperses Siliciumdioxid | 1,00 % |
| Natriumstärkeglykolat | 5 % |
| Natriumlaurylsulfat | 0,75 % |

| | Schuß 1 | Schuß 2 | Schuß 3 | Schuß 4 | Kopf |
|---|---|---|---|---|---|
| Temp. (°C) | 80 | 120 | 120 | 130 | 145 |

Die Prüfung der Wirkstoff-Freisetzung ergab, daß nach 30 min 98 % des Wirkstoffes freigesetzt war.

### Beispiele 11 bis 13

### Vitamim-B-Komplex Tabletten

Extrusion / Kalandrierung - Allgemeines:
Extrudertyp: Zweischneckenextruder
Anzahl der Schüsse: 4 + Kopf
Tablettenform, rund, Linsenform, ca. 240 mg schwer
Massenfluß: 20 - 25 kg/h

Die Mengenangaben beziehen sich jeweils auf Gew.-%.

### Beispiel 11

| | |
|---|---|
| Thiamin-Mononitrat | 0,572 % |
| Riboflavin | 0,704 % |
| Pyridoxin-HCl | 0,748 % |
| Cyanocobalamin (0,1 %ige Maltodextrineinbettung) | 1,32 % |
| Calcium-D-Pantothenat | 2,64 % |
| Nicotinsäure | 7,26 % |
| Folsäure | 0,066 % |
| Biotin | 0,02 % |
| Isomalt | 31,67 % |
| Hydroxypropylcellulose (Klucel® EF) | 50,00 % |
| Lecithin | 5,00 % |

| | Schuß 1 | Schuß 2 | Schuß 3 | Schuß 4 | Kopf |
|---|---|---|---|---|---|
| Temp. (°C) | 80 | 100 | 105 | 105 | 110 |

### Beispiel 12

| | |
|---|---|
| Thiamin-Mononitrat | 0,65% |
| Riboflavin | 0,8 % |
| Pyridoxin-HCl | 0,85 % |
| Cyanocobalamin (0,1 %ige Maltodrextrineinbettung) | 1,5 % |
| Calcium-D-Pantothenat | 3,00 % |
| Nicotinsäure | 8,25 % |
| Folsäure | 0,075 % |
| Biotin | 0,015 % |
| Isomalt | 45,86% |
| Hydroxypropylcellulose | 35,00 % |
| Lecithin | 4 % |

| | Schuß 1 | Schuß 2 | Schuß 3 | Schuß 4 | Kopf |
|---|---|---|---|---|---|
| Temp. (°C) | 80 | 90 | 90 | 100 | 115 |

### Beispiel 13

| | |
|---|---|
| Thiamin-Mononitrat | 0,3575 % |
| Riboflavin | 0,44 % |
| Pyridoxin-HCl | 0,4583 % |
| Calcium-D-Pantothenat | 1,65 % |
| Nicotinamid | 4,537 % |
| Biotin | 0,0178 % |
| Isomalt | 74,538 % |
| Hydroxypropylmethylcellulose | 10,00 % |
| Kollidon K 30 | 5 % |
| Lecithin | 3 % |

| | Schuß 1 | Schuß 2 | Schuß 3 | Schuß 4 | Kopf |
|---|---|---|---|---|---|
| Temp. (°C) | 80 | 120 | 130 | 133 | 133 |

## Patentansprüche

1. Feste Arzneiformen, erhältlich durch Extrusion mit anschließender Formgebung einer lösungsmittelfreien Schmelze, enthaltend neben einem oder mehreren Wirkstoffen
A) 2 bis 90 Gew.-% eines thermoplastisch verarbeitbaren, wasserlöslichen Polymers,
B) 5 bis 89,9 Gew.-% Isomalt, und
C) 0 bis 5 Gew.-% Lecithin,
wobei die Summe aller Inhaltsstoffe gleich 100 Gew.-% sein soll.

2. Arzneiformen nach Anspruch 1, enthaltend
A) 10 bis 90 Gew.-% eines thermoplastisch verarbeitbaren, wasserlöslichen Polymers,
B) 5 bis 89,9 Gew.-% Isomalt, und
C) 0 bis 5 Gew.-% Lecithin,
wobei die Summe aller Inhaltsstoffe gleich 100 Gew.-% sein soll.

3. Feste Arzneiformen nach Anspruch 1 oder 2, enthaltend als Polymer A) ein Homo- oder Copolymer eines N-Vinyllactams.

4. Feste Arzneiformen nach Anspruch 3, enthaltend ein Copolymer aus N-Vinylpyrrolidon und Vinylacetat.

5. Feste Arzneiformen nach Anspruch 1 oder 2, enthaltend als Polymer A) eine Hydroxypropylcellulose.

6. Feste Arzneiform nach einem der Ansprüche 1 bis 4, enthaltend als Wirkstoff Verapamil oder dessen physiologisch verträgliche Salze.

7. Feste Arzneiform nach einem der Ansprüche 1 bis 5 enthaltend zusätzlich übliche pharmazeutische Hilfsstoffe.

8. Verfahren zur Herstellung von Arzneiformen gemäß einem der Ansprüche 1 bis 6, **dadurch gekennzeichnet, daß** man die Komponenten bei Temperaturen von 50 bis 180°C zu einer Schmelze verarbeitet.

## Claims

1. A solid drug form obtainable by extrusion with subsequent shaping of a solvent-free melt, comprising, besides one or more active ingredients,
A) 2-90% by weight of a melt-processable, water-soluble polymer,
B) 5-89.9% by weight of isomalt, and
C) 0-5% by weight of lecithin,
where the total of all the ingredients is to be equal to 100% by weight.

2. A drug form as claimed in claim 1, comprising
A) 10-90% by weight of a melt-processable, water-soluble polymer,
B) 5-89.9% by weight of isomalt, and
C) 0-5% by weight of lecithin,
where the total of all the ingredients is to be equal to 100% by weight.

3. A solid drug form as claimed in claim 1 or 2, comprising as polymer A) a homo- or copolymer of an N-vinyllactam.

4. A solid drug form as claimed in claim 3, comprising a copolymer of N-vinylpyrrolidone and vinyl acetate.

5. A solid drug form as claimed in claim 1 or 2, comprising a hydroxypropylcellulose as polymer A).

6. A solid drug form as claimed in any of claims 1 to 4, comprising as active ingredient verapamil or its physiologically tolerated salts.

7. A solid drug form as claimed in any of claims 1 to 5, comprising in addition conventional pharmaceutical ancillary substances.

8. A process for producing drug forms as claimed in any of claims 1 to 6, which comprises converting the components into a melt at from 50 to 180°C.

## Revendications

1. Formes médicamenteuses solides obtenues par extrusion, suivie d'un façonnage, d'une masse fondue exempte de solvant contenant, avec une ou plusieurs substances actives,
A) 2 à 90 % en poids d'un polymère soluble dans l'eau et apte au travail thermoplastique,
B) 5 à 89,9 % en poids d'isomalt et
C) 0 à 5 % en poids de lécithine,
la somme de tous les constituants représentant 100 % en poids.

2. Formes médicamenteuses selon la revendication 1, contenant
A) 10 à 90 % en poids d'un polymère soluble dans l'eau et apte au travail thermoplastique,
B) 5 à 89,9 % en poids d'isomalt et
C) 0 à 5 % en poids de lécithine,
la somme de tous les composants représentant 100 % en poids.

3. Formes médicamenteuses solides selon la revendication 1 ou 2 contenant, en tant que polymère A) un homo- ou co-polymère d'un N-vinyllactame.

4. Formes médicamenteuses solides selon la revendication 3, contenant un copolymère de la N-vinylpyrrolidone et de l'acétate de vinyle.

5. Formes médicamenteuses solides selon la revendication 1 ou 2, contenant en tant que polymère A) une hydroxypropylcellulose.

6. Forme médicamenteuse solide selon l'une des revendications 1 à 4 contenant en tant que substance active du Verapamil ou ses sels convenant pour les applications pharmaceutiques.

7. Forme médicamenteuse solide selon l'une des revendications 1 à 5, contenant en outre des produits auxiliaires pharmaceutiques usuels.

8. Procédé pour la préparation de formes médicamenteuses selon l'une des revendications 1 à 6 **caractérisé par le fait que** l'on met les composants à l'état de masse fondue à des températures de 50 à 180° C.
